Europäisches Patentamt

European Patent Office

Office européen des brevets

(19)

(11) Publication number: **0 188 903**
**A1**

(12) # EUROPEAN PATENT APPLICATION

(21) Application number: **85309317.7**

(22) Date of filing: **20.12.85**

(51) Int. Cl.⁴: **C 12 Q 1/70**

---

(30) Priority: **28.12.84 US 687166**

(43) Date of publication of application: **30.07.86**
**Bulletin 86/31**

(84) Designated Contracting States: **AT BE CH DE FR GB IT LI LU NL SE**

(71) Applicant: **THE UNITED STATES OF AMERICA as represented by the Secretary UNITED STATES DEPARTMENT OF COMMERCE, 5285 Port Royal Road, Springfield, VA 22161 (US)**

(72) Inventor: **Harper, Mary E., 10232 Hatherleigh Drive Bethesda, Maryland 20814 (US)**
Inventor: **Wong-Staal, Flossie, 8418 Harker Drive Potomac, Maryland 20854 (US)**
Inventor: **Gallo, Robert C., 8513 Thornden Terrace Bethesda, Maryland 20834 (US)**

(74) Representative: **Daley, Michael John et al, F.J. CLEVELAND & COMPANY 40/43 Chancery Lane, London, WC2A 1JQ (GB)**

---

(54) **In situ detection of human T-cell leukemia virus type III.**

(57) In situ hybridization method for the detection of viral RNA or DNA, involving the hybridization of a tissue sample with an ³⁵S-labeled HTLV-III RNA probe. The probe detects HTLV-III in primary, uncultured tissue samples, is highly sensitive, and is relatively fast acting. The RNA probe may be synthesized from cloned HTLV-III DNA inserted into a suitable transcription vector.

EP 0 188 903 A1

## Background of the Invention

The present invention involves an in situ hybridization method useful for detecting rare cells (low abundancy, i.e. less than 100 copies per cell) expressing HTLV-III RNA in primary, uncultured tissue samples taken from infected individuals. This method significantly increases the sensitivity of detection over other known methods. For example, the RNA probe of this invention allows high efficiency hybridization and greater autoradiographic efficiency over conventional DNA probes labelled by nick translation. These new probes exhibiting high specific activity are generated by new strand synthesis involving high activity precursors. The resulting asymmetric probe (single strand) results in high efficiency hybridization in part due to elimination of probe rearrangement. Furthermore, ribonuclease synthesis on non-hybridized single-stranded probes result in lower levels of non-specific binding.

Additionally, incorporation of a $^{35}S$ label enhances autoradiographic efficiency over $^{3}M$ or $^{125}I$ labelled probes, permitting reduction or elimination of background labelling. Also, the half-life of decay (90 days) allows use of the labelled RNA for longer time periods.

The acquired immunodeficiency syndrome (AIDS) presents a severe, unexplained immune deficiency that involves reduction in the number of helper T lymphocytes (OKT4+). The disease is usually accompanied by multiple opportunistic infections and/or malignancies, the latter predominantly of the Kaposi's sarcoma type. In the prodromal stage (previously called preAIDS, now referred to as ARC or AIDS-related complex), other clinical manifestations may occur, most frequently unexplained chronic lymphadenopathy or leukopenia involving helper T lymphocytes.

It has been shown that the newly discovered human retrovirus, human T-cell leukemia (lymphotropic) virus type III (HTLV-III) is central in the development of AIDS. This virus shares many biological and physiochemical properties with HTLV types I and II, including a similar genomic organization, trans-activation activity of a virally-encoded polypeptide, tropism for T-lymphocytes, and distant nucleic acid homology. Multiple lines of evidence have implicated HTLV-III as the causative agent of AIDS. These include isolation of HTLV-III virus from the majority of patients with AIDS or ARC as well as from a significant number of individuals at risk for AIDS, and detection of antibodies reactive against HTLV-III antigens in almost all AIDS and ARC patients as well as hemophiliac AIDS cases, children with AIDS, and transfusion-associated, donor-recipient AIDS cases.

It is now possible to molecularly clone and analyze cDNA to HTLV-III genomic RNA, the unintegrated linear (replicative intermediate) form of HTLV-III, and full-length integrated proviral DNA forms of HTLV-III. These studies have shown that the HTLV-III genome is approximately 10 kilobases in length and lacks nucleic acid sequences present in normal human DNA. Using these clones for Southern blot analysis of fresh tissue specimens, HTLV-III viral DNA was detected at low levels in fresh lymphoid tissue from a number, albeit a minority, of patients with AIDS or ARC.

At the same time, in situ hybridization experiments were initiated to detect HTLV-III viral RNA in fresh tissue from AIDS or ARC patients. In situ hybridization offers several advantages as a screening method for HTLV-III sequences. The method is direct, quantitative, and requires very low quantities of tissue. Furthermore, information may be gained concerning the type(s) of cells infected with and expressing HTLV-III and the tissue distribution of HTLV-III sequences, as well as the distribution of cells expressing HTLV-III

- 3 -                    0188903

sequences within a particular tissue. Previous methods for RNA in situ hybridization exhibited relatively low sensitivity and could only detect RNA present at relatively high abundancy. It was therefore necessary to develop new methodology for in situ hydridization in order to detect HTLV-III RNA in primary tissue.

## Utility Statement

The present invention has specific application as a short diagnostic assay for detection of HTLV-III in primary, uncultured cells. Furthermore, this assay allows determination of a treatment's progress or success throughout and after treatment protocols.

The highly sensitive in situ hybridization method may also be applied to the detection of messenger RNA (mRNA) transcribed from genes important in transformation and/or tumor progression. For example, in situ hybridization may detect cells with increased mRNA expression of particular oncogenes which may signify an altered status. Such results, may for example, be used in staging malignancies, assaying therapeutic regimens, or detecting remission and relapse.

## Summary of the Invention

The in situ hybridization method for detection of viral RNA or DNA is briefly as follows. Primary, uncultured cells are placed onto microscope slides by one of two ways. Tissues from which cell suspensions can easily be made are centrifuged through ficoll-hypaque to isolate mononuclear cells. A suspension of these cells is then centrifuged onto the slides. Solid tissues are frozen and sections are cut using a cryostat. These sections are mounted on microscope slides. In either case, slides are fixed in paraformaldehyde for one minute and then stored in ethanol for periods extending up to six months or longer. Immediately before hybridization, slides are pretreated with several treatments to block reactive groups. Preparations are hybridized with a $^{35}$S-labeled HTLV-III RNA probe for 3 hours followed by a 2 hour rinsing procedure. The slides are coated with a photographic emulsion, allowed to expose for 2 days, developed for visualization of silver grains, stained with Wright stain, and analyzed by light microscopy.

In the above described in vitro process, which is more properly an assay, the RNA probe provides the basis for improvement over the known detection procedures. This probe detects HTLV-III in primary, uncultured tissue samples, is highly sensitive, and is relatively fast-acting (typically two days). The RNA probe is synthesized from cloned HTLV-III DNA inserted into a suitable transcription vector.

Specific Disclosure

Preparation of cells. Heparinized peripheral blood obtained from AIDS patients or normal individuals is centrifuged through Lymphocyte Separation Medium (Litton Bionetics) for separation of mononuclear cells. Cells are rinsed twice in RPMI 1640 media and resuspended at $10^6$ cells/ml in media containing 10% fetal calf serum. These cells are then deposited on microscope slides (previously cleaned with 70% ethanol) by cytocentrifugation of 200 µl cell suspension per slide. Slides are air-dried for 5 min. then fixed to the slide. For this, slides are immersed in 4% paraformaldehyde (Baker) in PBS (Ca, Mg-free) for 1 min. only, then transferred to 70% ethanol. Preparations are stored at 4°C until used for hybridization.

Preparation of $^{35}$S-labeled RNA Probes. pSP64-R3 (R3) consists of the 9.5 kb HTLV-III insert of clone BH10 inserted 3'-5' on transcription vector pSP64 (commercially obtained from Promega Biotech). R3 DNA is digested with Eco Rl, (which cuts midway in the viral genome) in order to generate linear DNA template molecules. These DNA molecules are phenol-chloroform extracted and ethanol precipitated before transcription. One microgram of R3 template DNA is transcribed in a 10 µl reaction containing 25 µM $^{35}$S-UTP (1000 Ci/mmol; New England

Nuclear), 500 μM ATP, CTP and GTP, 40 mM Tris-HCl (pH7.5), 6 mM $MgCl_2$, 2 mM spermidine, 20 mM NaCl, 10 mM DTT, 15 units RNasin (Promega Biotec), and 15 units SP6 polymerase (New England Nuclear). After incubation at 40°C for 30 min., 15 additional units of SP6 polymerase are added and unlabeled UTP is added to a final concentration of 500 μM. The reaction is allowed to continue for 30 additional minutes at 40°C. The DNA template is then digested by 20 μg/ml DNase (Worthington) at 37°C for 10 min., and the $^{35}$S-RNA purified according to Johnson and Johnson, 198). Typically, 70-90% of $^{35}$S-UTP is incorporated using this procedure. Purified RNA was stored in aliquots at -90C until used.

In situ hybridization. Slide preparations are rinsed briefly in 2XSSC, (a standard salt solution of chloride and citrate) and acetylated in acetic anhydride-triethanolamine, pH 8.0. The slides are then rinsed briefly in 2XSSC followed by PBS (Ca, Mg-free), and immersed in 0.1 M Tris-HCl (pH 7.0), 0.1 M glycine for 30 min. Slides are rinsed in 2XSSC and placed in 50% formamide-2XSSC at 55°C immediately before application of the RNA probe. The hybridization mixture contained $10^5$ cpm/μl $^{35}$S-RNA probe. 50% formamide, 2XSSC, 10 mM DTT, 1 ml/ml sheared salmon spern DNA, 1 ml/ml E. coli tRNA, and 2 ml/ml bovine serum albumin. The mixture is denatured by heating at 90°C for 15 min. and was then cooled to 55°C. Excess solution is removed from slides and 10 1 of hybridization mix is applied per slide. Coverslips (18 x 18 mm) are mounted with rubber cement and hybridization is carried out at 50°C for about 3 hr. The slides are then rinsed thoroughly in 50% formamide-2XSSC at 52°C, followed by several rinses in 2XSSC. Ribonuclease treatment with 100 μg/ml RNase A (Sigma) in combination with 1 μg/ml RNase $T_1$ (Boehringer) in 2XSSC is carried out for 30 min. at 37°C. The slides are again rinsed in 50% formamide-2XSSC at 52°C, followed by rinsing in 2XSSC and dehydration in ethanol. Hybridized preparations are autoradiographed with NTB2 nuclear track emulsion (Eastman) diluted 1:1 with distilled water. After exposure for 2 days at $4^{O}$C, the slides are developed in Dektol (Eastman) and dried and stained with Wright stain (Harleco).

This method allows in situ detection of HTLV-III viral RNA in a very low percentage of cells (typically 0.019%) and in a short period of time (2-5 days). Primary cells from AIDS, ARC or high-risk individuals may be assayed without culture. The types of samples (primary cells) include peripheral blood, bone marrow, lymph node and spleen cells from which a cell suspension is obtained, and solid tissue such as brain, lung, liver, lymph node, and Kaposi's sarcoma.

The HTLV-III clone BH10, from which a 9.0 Kb viral insert is obtained, is available through the American Type Culture collection, accession No. 40125. This clone is maintained in the ATCC in the manner prescribed by the U.S. Patent and Trademark Office with regard to permanence and availability of the deposit for 30 years (plus 5 years after request for sample) and without restriction on public access. Furthermore, the description of this HTVL-III clone may be found in a United States Patent Application Wong-Staal et al, Serial No. 643,306, filed 22nd August 1984, and titled "Molecular Clones of the Genome of HTVL-III". The corresponding EPC Application is 85 305878.2.

In general, cloning the HTVL-III genome involved isolating unintegrated viral DNA after infection of H9-cells with concentrated HTVL-III virus and cloning this DNA in a lambda phage library to be screened with viral cDNA. The cell line H9/HTVL-III produces large quantities of HTVL-III virus and serves

- 7 -

as the principal producer cell line for immunological assays used to detect virus specific antigens and antibodies in AIDS sera. Cultures of H9/HTVL-III cells (infected cells) are grown and harvested, followed by extraction of low molecular weight DNA from the newly infected cells. This produces unintegrated viral DNA. Unintegrated linear DNA (provirus DNA) is then obtained, containing the entire HTVL-III genome, i.e. replication competent. This DNA is then digested in plasmid lambda of Wes lambda B to form clone lambda BH10.

In situ hybridization as used above, is hybridization to nucleic acid(s) still in the cell, as opposed to RNA or DNA that has been isolated and/or purified away from the cell.

Additionally, the radiolabelled probe may be labelled with $^3$H, $^{125}$I, $^{32}$P, or preferably, $^{35}$S.

CLAIMS

1. A process for the in situ detection of human T-cell leukemia virus type-III (HTLV-III) consisting essentially of contacting a test sample with a $^{35}$S-labelled HTLV-III RNA probe, covering said sample with a photographic emulsion and analyzing said sample by light microscopy.

2. The process of claim 1, wherein said test sample is primary tissue culture.

3. The process of claim 1, wherein said probe consists essentially of forming a DNA template by digesting $R^3$ DNA with EcoRI to form linear DNA template molecules, transcribing said DNA molecules with $^{35}$S-UTP to form a labelled DNA template; digesting said labelled DNA template with DNase, and obtaining $^{35}$S-RNA.

4. A process for the detection of lymphocytes expressing RNA specific for HTLV-III consisting essentially of contacting a $^{35}$S-labelled RNA probe with a test sample and analyzing said sample by light microscopy for HTLV-III labelled with $^{35}$S.

5. A method for the in situ detection of HTLV-III consisting essentially of treating a test tissue sample with a radiolabelled RNA probe and analyzing for said probe.

6. The method of claim 5, wherein said radiolabelled probe is labelled with one member of the group consisting of $^{3}$H, $^{125}$I, $^{32}$P, $^{35}$S.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | NATURE, vol. 312, no. 5990, 8th November 1984, pages 166-169, Reading, Berks, GB; B.H. HAHN et al.: "Molecular cloning and characterization of the HTLV-III virus associated with AIDS" * Whole article * | 1-6 | C 12 Q 1/70 |
| X | NATURE, vol. 312, no. 5996, December/January 1984/85, pages 757-760, London, GB; M. ALIZON et al.: "Molecular cloning of lymphadenopathy-associated virus" * Whole article * | 1-6 | |
| X | NATURE, vol. 312, no. 5996, December/January 1984/85, pages 760-763, London, GB; P.A. LUCIW et al.: "Molecular cloning of AIDS-associated virus" * Whole article * | 1-6 | TECHNICAL FIELDS SEARCHED (Int. Cl.4) C 12 Q C 12 N G 01 N |
| X,Y | SCIENCE, vol. 226, 7th December 1984, pages 1165-1171; G.M. SHAW et al.: "Molecular characterization of human T-cell leukemia (lymphotropic) virus type III in the Acquired Immune Deficiency Syndrome" * Whole article * | 1-6 | |

-/-

The present search report has been drawn up for all claims

| Place of search | Date of completion of the search | Examiner |
|---|---|---|
| THE HAGUE | 06-05-1986 | OSBORNE H.H. |

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 97, no. 17, 25th October 1982, page 381, no. 141585j, Columbus, Ohio, US; A. REIN et al.: "In situ hybridization: general infectivity assay for retroviruses", & J. VIROL. 1982, 43(3), 1055-60 * Whole abstract * | 1,5 | |
| Y | EP-A-0 124 124 (ENZO BIOCHEM INC.) * Abstract; examples 8,9 * | 1,4,5 | |
| P,Y | EP-A-0 135 108 (ROCKEFELLER UNIVERSITY) * Abstract; page 11, line 20 - page 12, line 15 * | 1,5,6 | |
| P,Y | EP-A-0 155 360 (J.A. WEBSTER, Jr.) * Page 22, line 21 - page 23, line 29; page 41, line 27 - page 42, line 8 * | 1,5,6 | TECHNICAL FIELDS SEARCHED (Int. Cl.4) |

The present search report has been drawn up for all claims

| Place of search | Date of completion of the search | Examiner |
|---|---|---|
| THE HAGUE | 06-05-1986 | OSBORNE H.H. |